# FASCICULE DE BREVET EUROPEEN

(11) **EP 0 437 390 B1**
(45) Date de publication et mention de la délivrance du brevet: **03.05.1995**
(21) Numéro de dépôt: 91400024.5
(22) Date de dépôt: 08.01.1991
(51) Int. Cl.: A61F 2/38

(54) **Pièce fémorale pour prothèse du genou**
Femurteil für eine Kniegelenkprothese
Femoral component for a knee prosthesis

(30) Priorité: 12.01.1990 FR 9000335
(43) Date de publication de la demande: 17.07.1991
(73) Titulaire: SOCIETE CIVILE D'INNOVATIONS TECHNOLOGIQUES, 77123 Noisy sur Ecole (FR)
(72) Inventeur: Pequignot, Michel, F-92140 Clamart (FR)
(74) Mandataire: CABINET BONNET-THIRION

(56) Documents cités:
- FR-A- 2 105 998
- FR-A- 2 142 028
- FR-A- 2 216 981
- US-A- 3 715 763
- US-A- 4 000 525

## Description

La présente invention concerne d'une manière générale les prothèses totales du genou, du type de celles mises en oeuvre dans le cas d'arthroses, ou gonarthroses, très évoluées.

Elle vise plus particulièrement la partie fémorale de ces prothèses.

Il s'agit, par exemple, d'une pièce comportant, d'un seul tenant, sous la forme générale d'une fourche globalement cintrée, d'une part, deux branches latérales, qui sont destinées à être chacune respectivement appliquées aux deux condyles du fémur à traiter, pour coopération avec les glènes du plateau tibial correspondant, et, d'autre part, une partie médiane, avec laquelle se raccordent les deux branches latérales précédentes, et qui présente, à la racine de celles-ci, une saignée largement adoucie formant la trochlée fémorale associée.

Cette pièce fémorale, dont la forme est ainsi relativement compliquée, et dont la tenue sur le fémur, après travail de celui-ci, se fait essentiellement par emboîtement, doit nécessairement être extrêmement rigide.

Il s'agit donc, d'une façon absolument générale à ce jour, et tel que décrit par exemple dans la demande de brevet français publiée sous le No 2.142.028, d'une pièce métallique, qui, réalisée par exemple par moulage, notamment à la cire perdue, met en oeuvre des métaux ou alliages métalliques choisis pour leur biocompatibilité, tels que par exemple un acier inoxydable à bas taux de carbone, un alliage au chrome nickel cobalt, ou du titane allié, et, plus particulièrement, du titane allié au vanadium.

L'une des difficultés dans la réalisation des prothèses totales du genou comportant une telle pièce fémorale tient notamment à ce que, à la différence des prothèses de type sphéroïde, où les surfaces frottantes en contact sont parfaitement concordantes, l'articulation à reconstituer nécessite une certaine discordance des surfaces frottantes correspondantes, notamment en raison de ce que, lors des mouvements de flexion-extension intervenant par exemple au cours d'une marche normale, le roulement des condyles sur les glènes s'accompagne d'un glissement sur ceux-ci.

Il importe donc que, entre la partie fémorale d'une prothèse totale du genou et la partie tibiale de celle-ci, il y ait une bonne adaptation au frottement.

Pour ce faire, la partie tibiale comporte usuellement une pièce de frottement en matière synthétique, en pratique du polyéthylène haute densité, sur laquelle sont formées les glènes du plateau tibial à reconstituer, et qui est rapportée sur une pièce de support, métallique, ancrée, elle, dans le tibia à équiper.

Malheureusement, il s'avère que le polyéthylène haute densité indispensable en l'espèce a tendance à devenir abrasif lorsqu'il se détériore.

Il en résulte que, suivant un cycle qui ne peut que s'accélérer, il devient agressif à l'égard de la pièce fémorale métallique qui est à son contact, notamment lorsque celle-ci est en acier inoxydable à bas taux de carbone ou en titane allié.

Il a donc été proposé d'appliquer aux surfaces frottantes de cette pièce fémorale, qui par ailleurs doivent être soigneusement polies, un traitement de surface destiné à leur conférer un certain durcissement superficiel.

S'agissant, par exemple, d'une pièce fémorale en titane allié, il a été proposé d'en assurer une nitruration par implantation ionique.

Une telle nitruration permet effectivement d'obtenir des duretés de surface et des coefficients de frottement très favorables.

Mais sa mise en oeuvre relève de techniques très complexes, et elle nécessite des soins extrêmes quant à la propreté à respecter pour les surfaces à traiter, toujours polluées par leur polissage préalable.

Elle est donc particulièrement coûteuse.

Enfin, et surtout, les épaisseurs effectivement traitées sont toujours très faibles, de l'ordre par exemple de deux microns.

Il en résulte la formation, en surface, d'une couche, qui est effectivement très dure, mais qui est également très fragile, et qui est notamment intempestivement susceptible d'un enfoncement dans le substrat métallique, plus tendre, sous-jacent.

Il a également été proposé de revêtir de céramique d'oxyde métallique les surfaces frottantes à traiter, par projection au plasma des oxydes métalliques correspondants.

Les dépôts obtenus, qui sont notoirement plus épais que les précédents, en atteignant par exemple une épaisseur de l'ordre de quelques dixièmes de millimètres, ont pour avantage d'être particulièrement durs et d'être susceptibles d'excellents coefficients de frottement.

Mais ils ont le triple inconvénient de devoir nécessairement être polis pour l'obtention de tels coefficients de frottement, d'être poreux, ce qui les rend de manière intempestive susceptibles de piéger n'importe quelle particule parasite, et en particulier celles résultant du polissage précédent, et de ne présenter qu'une adhérence limite vis-à-vis du substrat sous-jacent, au risque d'en voir une partie se détacher localement de ce substrat et engendrer ensuite une dégradation rapide et irréversible des surfaces frottantes en jeu.

Il n'est pas envisageable, par ailleurs, de réaliser, d'un seul tenant, en céramique, de manière économiquement exploitable, la pièce fémorale en cause, en raison, notamment, du prix propre des oxydes métalliques à mettre en oeuvre, du retrait à la cuisson des céramiques auxquelles ils donnent naissance, et du coût des usinages à appliquer ensuite à celles-ci.

La présente invention a d'une manière générale pour objet une disposition permettant cependant de mettre en oeuvre de la céramique pour une pièce fémorale pour prothèse totale du genou tout en surmontant ces difficultés.

De manière plus précise, elle a pour objet une pièce fémorale pour prothèse du genou présentent les caractéristiques de la revendication 1.

Le bandage, qui est dûment rapporté, à la manière d'une jante, sur la pièce fémorale à équiper, en étant convenablement assujetti à celle-ci, par exemple par sertissage ou collage, et qui se distingue en cela d'un simple revêtement de surface résultant d'un dépôt directement effectué in situ, permet avantageusement de minimiser la quantité de céramique à mettre en oeuvre, en la réduisant à la seule partie de cette pièce fémorale effectivement soumise au frottement.

Mais, réalisé en céramique massive, il ne présente pas les inconvénients, rappelés ci-dessus, d'un tel revêtement de surface.

Lorsque, de manière usuelle, la pièce fémorale à équiper est du genre comportant, sous la forme générale d'une fourche globalement cintrée, d'une part, deux branches latérales, qui sont destinées à être chacune respectivement appliquées aux deux condyles du fémur à traiter, pour coopération avec les glènes du plateau tibial correspondant, et, d'autre part, une partie médiane, avec laquelle se raccordent les branches latérales précédentes, chacune de ces branches latérales est suivant l'invention munie d'un bandage en céramique sur une partie au moins de sa longueur.

Il reste cependant la difficulté de réaliser un tel bandage en céramique, sa forme étant relativement compliquée, et nécessitant normalement un ajustage à effectuer avec une grande précision.

La présente description décrit divers procédés propres à permettre de réaliser de manière économiquement acceptable un tel bandage en céramique, sans avoir recours à des machines de rectification particulièrement onéreuses.

Un premier de ces procédés implique la découpe en diagonale d'un anneau aplati en céramique.

Par exemple, cet anneau aplati peut résulter de l'aplatissement à chaud sous charge d'un anneau initialement cylindrique, et, pour sa mise en forme, il est ensuite simplement disposé dans un moule réalisé en un matériau dont le coefficient de dilatation thermique est inférieur au sien, en tirant parti du différentiel existant alors entre les coefficients de dilatation thermique en jeu.

Quoi qu'il en soit, un tel procédé conduit avantageusement à l'obtention simultanée de deux ébauches de bandage, qu'il suffit ensuite d'usiner pour les polir et les effiler, et qui sont propres conjointement à l'équipement d'une même pièce fémorale.

Un deuxième procédé implique l'obtention individuelle d'une telle ébauche de bandage par engagement, à force et à chaud, d'une plaquette en céramique dans une rainure conformée au profil recherché.

En variante, un troisième procédé implique l'obtention d'une ébauche de bandage par insertion d'une plaquette en céramique entre les deux parties d'un moule et rapprochement relatif à chaud de celles-ci.

Dans l'un et l'autre cas, il suffit, ensuite, comme précédemment, d'usiner l'ébauche de bandage obtenue.

En pratique, ces divers procédés ont en commun de partir d'une pièce en céramique préexistante et de la conformer de manière à permettre son application à la pièce fémorale à équiper.

Enfin, la présente description décrit un procédé qui consiste, pour la réalisation de la partie fémorale d'une prothèse du genou, à former sous la forme d'une pièce distincte au moins un bandage en céramique et à rapporter ce bandage en céramique sur une pièce fémorale métallique adaptée elle-même à être rapportée sur le fémur à traiter.

L'objet de l'invention, ses caractéristiques et ses avantages, ressortiront d'ailleurs de la description qui va suivre, à titre d'exemple, en référence aux dessins schématiques annexés sur lesquels ;
la figure 1 est une vue en perspective d'une prothèse du genou mettant en oeuvre une pièce fémorale suivant l'invention ;
la figure 2 est, à échelle supérieure, une vue en perspective de cette seule pièce fémorale, avec, en place, l'un des deux bandages en céramique qu'elle comporte tandis que l'autre en est retiré ;
la figure 3 est une vue partielle en coupe transversale de cette pièce fémorale, suivant la ligne III-III de la figure 2 ;
la figure 4 est une vue partielle en coupe transversale analogue à celle de la figure 3, pour une variante de réalisation de la pièce fémorale suivant l'invention ;
les figures 5A, 5B, 5C, 5D, 5E et 5F sont des vues en plan ou en plan-coupe illustrant diverses phases successives d'un procédé propre à la réalisation d'un tel bandage en céramique ;
les figures 6A, 6B sont des vues en perspective illustrant deux phases successives d'un autre procédé propre à l'obtention d'un tel bandage en céramique ;
la figure 7 est une vue en perpsective d'un moule mis en oeuvre suivant un autre procédé pour le formage d'un tel bandage en céramique, pour la position de fermeture, à vide, de ce moule ;
la figure 8 est une vue en perspective analogue à celle de la figure 7, pour la position d'ouverture du moule concerné ;
la figure 9 est, à échelle supérieure, une vue partielle en coupe transvrersale d'une des parties de ce moule, suivant la ligne IX-IX de la figure 8 ;
la figure 10 est, à l'échelle des figures 7 et 8, une vue en perspective d'une plaquette en céramique à former ;
les figures 11A, 11B, 11C sont, à échelle inférieure, des vues en élévation du moule, pour diverses phases successives d'intervention de celui-ci ;
la figure 12 est une vue en perspective de l'ébauche de bandage obtenue au terme du formage correspondant ;
la figure 13 est une vue en perspective qui, analogue à celle de la figure 12, se rapporte à une variante de mise en oeuvre;
la figure 14 est une vue en perspective éclatée qui, analogue à celle de la figure 2, se rapporte à une variante de réalisation.

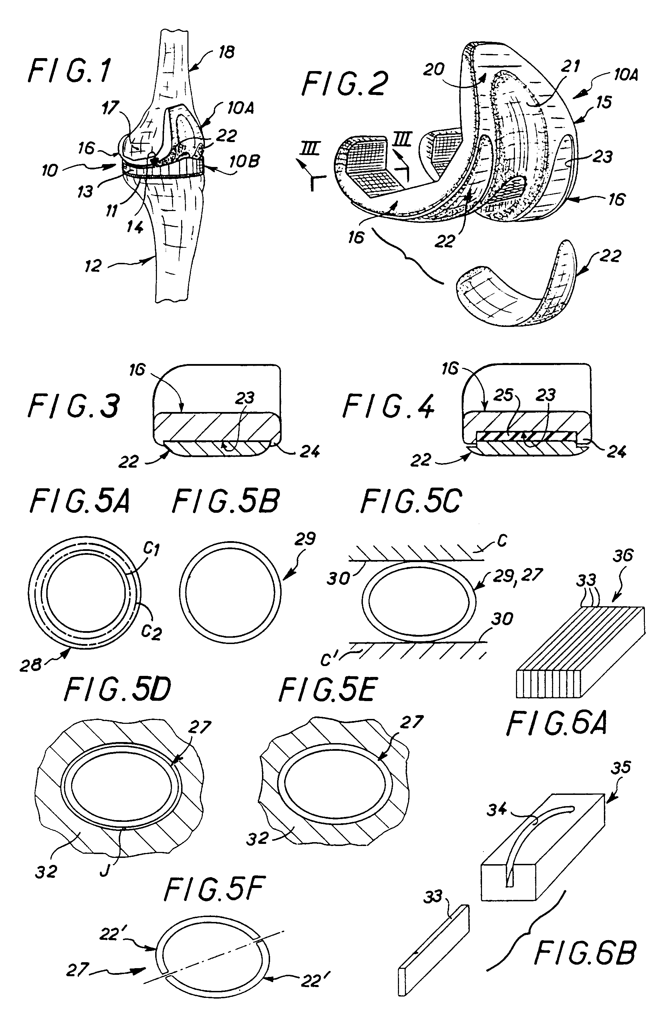

Tel qu'illustré sur la figure 1, une prothèse totale de genou 10 comporte, globalement, une partie fémorale 10A et une partie tibiale 10B.

Seule la partie fémorale 10A est en cause ici.

S'agissant de la partie tibiale 10B, il suffira d'indiquer qu'elle se compose, usuellement, et tel que représenté, de deux pièces, à savoir une pièce de support 11, qui est en général métallique, et qui est ancrée dans le tibia 12 à équiper, en bout de celui-ci, et une pièce de frottement 13, qui est rapportée sur la pièce de support 11, et qui, réalisée, elle, en matière synthétique, et, en pratique, en polyéthylène haute densité, forme, en surface, le plateau tibial 14 correspondant, avec les surfaces articulaires, ou glènes, non visibles sur la figure, avec lesquelles doit coopérer en roulement et glissement la partie fémorale 10A.

En pratique, cette partie fémorale 10A se réduit à une pièce fémorale unique 15.

Suivant des dispositions qui, bien connues en elles-mêmes, et ne relevant pas de la présente invention, ne seront pas décrites ici, cette pièce fémorale 15, qui est en pratique une pièce métallique, est adaptée à être rapportée sur le fémur 18 à traiter, en bout de celui-ci, par exemple à l'aide de vis.

Dans la forme de réalisation représentée, elle comporte, sous la forme générale d'une fourche globalement cintrée autour d'un axe horizontal, d'une part, deux branches latérales 16, qui sont destinées à être chacune respectivement appliquées aux deux condyles 17 du fémur 18 à traiter, pour coopération avec les glènes du plateau tibial 14 correspondant, et, d'autre part, une partie médiane 20, avec laquelle se raccordent, globalement en V, les branches latérales 16 précédentes, et qui, à la racine de celles-ci, présente une saignée 21, largement adoucie, formant la trochlée fémorale associée.

Une partie au moins de la surface frottante de la pièce fémorale 15 est munie d'un bandage en céramique.

Dans la forme de réalisation représentée sur la figure 2, il en est ainsi pour ses seules branches latérales 16.

Chacune de ces branches latérales 16 est donc munie, sur une partie au moins de sa longueur, et, en pratique, sur la totalité de sa longueur utile, d'un bandage en céramique 22.

Les bandages en céramique 22 dont est ainsi équipée la pièce fémorale 15 forment des pièces en céramique massive qui, initialement distinctes de cette pièce fémorale 15, sont dûment rapportées sur celle-ci, en lui étant convenablement assujetties.

Par exemple, figures 1 à 3, chacun des bandages en céramique 22 est engagé dans un logement 23 prévu à cet effet sur la branche latérale 16 correspondante, et il est assujetti par sertissage à celle-ci, par rabattement, contre lui, des bords 24 de ce logement 23.

En variante, figure 4, il est procédé par collage.

Dans la forme de réalisation représentée sur la figure 4, des moyens d'amortissement sont en outre interposés entre une branche latérale 16 de la pièce fémorale 15 et le bandage en céramique 22 qu'elle porte.

Par exemple, et tel que représenté, il s'agit d'un matelas 25 en matériau élastique.

Ce matelas 25 est engagé dans le logement 23 correspondant, en étant rapporté par collage sur le fond de celui-ci.

Le bandage en céramique 22 peut lui être ensuite rapporté par collage.

Dans la forme de réalisation représentée, le bandage en céramique 22 déborde du contour du logement 23, et surplombe, avec jeu, les bords 24 de celui-ci.

Mais il n'en est pas obligatoirement ainsi.

Quoi qu'il en soit, chacun des bandages en céramique 22 mis en oeuvre se présente sous la forme générale d'une lamelle qui, globalement cintrée autour d'un axe orthogonal à sa plus grande dimension, va en diminuant de largeur, de manière dissymétrique, de l'arrière vers l'avant.

En bref, les bandages en céramique 22 sont effilés du côté de la saignée 21 formant la trochlée fémorale, et ils sont globalement symétriques l'un de l'autre par rapport à l'axe de celle-ci.

Pour la constitution de ces bandages en céramique 22, on choisit de préférence une céramique d'oxyde métallique dont les qualités de biocompatibilité, de résistance mécanique, et de coefficient de frottement vis-à-vis du polyéthylène haute densité, sont optimales.

A titre indicatif, il sera indiqué ici que la zircone, et plus précisément la zircone stabilisée à 3 % d'oxyde d'yttrium, donne à cet égard toute satisfaction, en raison, notamment, d'une grande finesse de grain susceptible de permettre un polissage particulièrement poussé.

Mais l'alumine, dont le grain est plus gros, peut également convenir.

De préférence, cependant, les bandages en céramique 22 mis en oeuvre suivant l'invention sont en zircone.

Longitudinalement, la forme des bandages en céramique 22 s'apparente à celle de patins, leur partie avant étant plus relevée, et plus cintrée, que leur partie arrière.

Partant de l'observation que, mis tête-bêche, ces bandages en céramique 22 forment globalement un anneau aplati, un premier procédé propre, suivant l'invention, à la réalisation de ces bandages en céramique 22 implique, tel que schématisé par un trait interrompu à la figure 5F, la découpe en diagonale d'un anneau aplati 27 en céramique, ce premier procédé conduisant ainsi avantageusement à l'obtention simultanée de deux ébauches de bandage 22′.

De manière plus précise, il est, dans ce cas, procédé comme suit.

Tel que schématisé à la figure 5A, le produit de départ est un anneau en céramique 28 obtenu, de manière usuelle, par cuisson d'une ébauche réalisée par pressage isostatique.

Compte tenu des retraits résultant de cette cuisson, cet anneau en céramique 28 n'est pas rigoureusement cylindrique.

Il est donc procédé ensuite à son réalésage, tant intérieurement, suivant le cylindre C1 schématisé en traits interrompus sur la figure 5A, qu'extérieurement, suivant le cylindre C2, ce qui conduit à l'obtention d'un anneau en céramique 29 parfaitement cylindrique, figure 5B.

S'agissant d'un anneau en zircone, il est tiré ensuite parti de la propriété qu'a une telle céramique de se déformer sous charge à haute température.

Tel que schématisé à la figure 5C, il est en effet alors procédé à l'aplatissement à chaud sous charge de l'anneau initialement cylindrique 29.

A titre indicatif, il peut être indiqué que, pour l'obtention de l'aplatissement recherché, il suffit par exemple d'appliquer à l'anneau initialement cylindrique 29 une charge de 3 kg et de le porter à une température de l'ordre de 1500°C.

Par exemple, le cycle thermique correspondant peut impliquer une montée en température de 5°C/mn jusqu'à 1000°C et de 10°C/mn au-delà, puis un palier de 15 mn à la température de 1500°C atteinte, avant une descente en température symétrique de la montée.

En pratique, l'affaissement recherché débute vers 1300°C, et il est pratiquement achevé à 1450°C.

En pratique, également, cet affaissement conduit à un aplatissement de l'anneau cylindrique 29 de l'ordre de 10 % suivant son petit axe, plus marqué dans ses zones de contact avec la charge que dans ses zones éloignées de celle-ci.

Dans la forme de réalisation illustrée, figure 5C, la charge C est appliquée suivant une génératrice de cet anneau cylindrique 29, avec contrebutée C′ de celui-ci suivant sa génératrice opposée.

Mais, en variante, elle peut être répartie entre plusieurs génératrices de cet anneau cylindrique 29, avec une répartition symétrique de la contrebutée correspondante.

Quoi qu'il en soit, du fait de son affaissement, l'anneau initialement cylindrique 29 conduit à un anneau aplati 27 dont le contour s'apparente d'une manière générale à celui d'une ellipse.

Pour l'obtention de la forme définitive souhaitée pour les bandages en céramique 22, il est procédé, ensuite, à une mise en forme de cet anneau aplati 27 avant sa découpe.

Suivant l'invention, il est procédé, pour ce faire, par expansion.

Plus précisément, l'anneau aplati 27 est disposé dans un moule 32 réalisé en un matériau réfractaire dont le coefficient de dilatation thermique est inférieur au sien, et l'ensemble est porté à une température suffisante pour que, eu égard aux coefficients de dilatation thermique en question, et au jeu périphérique J existant initialement entre l'anneau aplati 27 et le moule 32, cet anneau aplati 27 vienne s'appliquer contre ce moule 32.

Par exemple, s'agissant d'un anneau aplati 27 réalisé en zircone, dont le coefficient de dilatation est de l'ordre de 1,7.10⁻⁵ par °C le moule 32 est réalisé en alumine, dont le coefficient de dilatation est de l'ordre de 0,7.10⁻⁵ par °C.

L'anneau aplati 27 est placé à froid dans ce moule 32, et l'ensemble est porté à 1500°C.

A cette température, la diminution linéaire de la zircone est d'environ 1 mm supérieure à celle de l'alumine pour une dimension de 60 mm.

Cette différence d'expansion conduit l'anneau aplati 27 à se plaquer, de lui-même, de manière parfaite, contre le moule 32, tel que schématisé à la figure 5E, et donc à épouser la forme de ce moule 32.

Il est procédé ensuite à l'enlèvement à froid de l'anneau aplati 27 ainsi mis en forme, puis à sa découpe.

Par exemple, les ébauches de bandage 22′ alors obtenues peuvent avoir une épaisseur de l'ordre de 1 à 2 mm.

Bien entendu, les diverses valeurs numériques données ci-dessus ne l'ont été qu'à titre d'exemples indicatifs.

Après leur obtention, les ébauches de bandage 22′ sont usinées, de manière, notamment, à être effilées dans leur partie avant.

Bien entendu, les bandages en céramique 22 correspondants sont polis.

Leur polissage peut se faire avant leur mise en place sur les branches latérales 16 de la pièce fémorale 15 qu'ils doivent équiper, ou après cette mise en place.

Quoi qu'il en soit, ce polissage se fait suivant les techniques, à pâte diamantée par exemple, usuelles en la matière.

Tel que schématisé sur les figures 6A, 6B, un autre procédé implique, pour la réalisation d'une ébauche de bandage 22′, l'engagement, à force et à chaud, d'une plaquette en céramique 33 dans une rainure 34 conformée au profil recherché pour une telle ébauche de bandage 22′.

Cette rainure 34 est en pratique usinée en conséquence dans un conformateur en matériau réfractaire 35.

Par exemple, et tel que schématisé à la figure 6A, la plaquette en céramique 33 résulte de la découpe préalable d'un bloc en céramique initialement parallélépipédique 36.

Quoi qu'il en soit, pour les ébauches de bandage 22′ ainsi obtenues par conformation, il est procédé ensuite comme précédemment.

Suivant un troisième procédé, il est mis en oeuvre, pour la réalisation d'une ébauche de bandage 22′, un formage à chaud, sous charge, en tirant parti de la capacité de fluage que présente la céramique au-delà d'une certaine température.

L'expérience montre, en effet, et les essais confirment, qu'il est possible ainsi d'avoir très facilement, et de manière reproductible, l'ébauche de bandage 22′ recherchée.

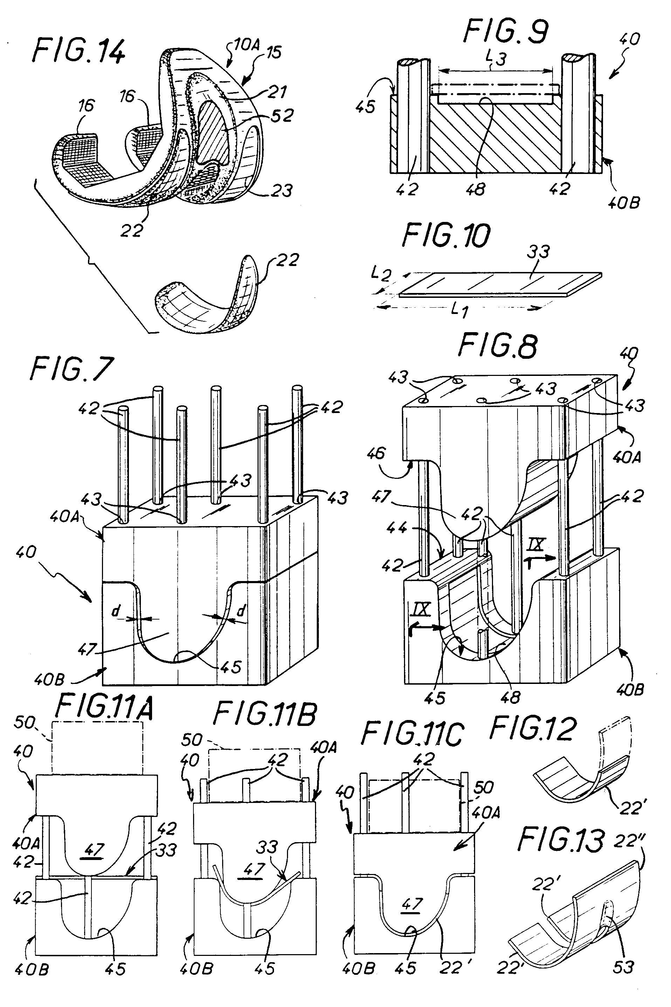

Plus précisément, suivant ce troisième procédé, il est mis en oeuvre un moule 40, figure 7 à 9.

Il s'agit d'un moule formé de deux parties 40A, 40B.

Dans la forme de mise en oeuvre représentée, ces deux parties 40A, 40B sont disposées l'une au-dessus de l'autre, et la partie inférieure 40B porte des guides 42 sur laquelle la partie supérieure 40A est montée coulissante à la faveur de perçages 43 qui la traversent à cet effet de part en part.

Par exemple, lorsque, tel que représenté, l'ensemble forme, globalement, un bloc parallélépipédique, un alignement de guides 42 peut ainsi être prévu le long de chacun des bords de plus grande longueur de ce bloc.

Dans la forme de réalisation représentée, qui concerne un prototype d'essai, trois guides 42 sont prévus le long de chacun de ces bords, et il s'agit de colonnettes de section transversale circulaire.

Bien entendu, leur nombre peut être réduit et/ou leur implantation être différente.

Pour la définition de la cavité de moulage nécessaire, l'une quelconque des parties 40A, 40B du moule 40, et il s'agit, en pratique, dans la forme de mise en oeuvre représentée, de la partie 40B, présente, en creux, dans la zone médiane d'un épaulement 44, un évidement 45, qui est globalement au contour du bandage en céramique 22 recherché.

Conjointement, l'autre des parties 40A, 40B de ce moule 40, et il s'agit donc, en l'espèce, dans la forme de réalisation représentée, de la partie 40A, présente, en relief, dans la zone médiane d'un épaulement 46, un bossage 47, qui, complémentaire de l'évidement 45 précédent, est apte à s'engager dans celui-ci.

Dans la forme de mise en oeuvre représentée, l'évidement 45 de la partie 40B et le bossage 47 de la partie 40A sont définis par des génératrices parallèles, à la manière de parois cylindriques, et ils s'étendent transversalement sur toute la largeur des parties 40A, 40B qu'ils concernent.

En pratique, les épaulements 44, 46 dans la zone médiane desquels interviennent cet évidement 45 et ce bossage 47 s'étendent globalement perpendiculairement aux guides 42.

De préférence, et ainsi qu'il est visible à la figure 7, il est prévu, entre l'évidement 45 et le bossage 47, le long de leurs faces latérales, et pour des raisons qui seront exposées ultérieurement, une dépouille d.

De préférence, également, et ainsi qu'il est visible sur les figures 8 et 9, la partie 40A, 40B concernée par l'évidement 45, en l'espèce la partie 40B, présente, transversalement, en creux, tout au long de cet évidement 45, dans la zone médiane de celui-ci, entre les guides 42, pour des raisons qui seront également exposées ultérieurement, un dégagement 48.

En pratique, il s'agit d'un dégagement à fond plat et bords droits, de relativement faible épaisseur.

Ainsi qu'on le notera, ce dégagement 48 ne s'étend pas sur l'épaulement 44 correspondant.

En pratique, les deux parties 40A, 40B du moule 40 ainsi constitué sont en graphite.

Conjointement, les guides 42 qu'elles comportent peuvent par exemple être en molybdène.

La réalisation d'un bandage en céramique 22 propre à l'équipement de la pièce fémorale 15 implique, tout d'abord, l'obtention d'une ébauche de bandage 22′, par insertion d'une plaquette en céramique 33 entre les deux parties 40A, 40B du moule 40 et rapprochement relatif à chaud de celles-ci.

Comme décrit précédemment, la plaquette en céramique 33 peut par exemple résulter de la découpe préalable d'un bloc en céramique, et, par exemple, d'un bloc en zircone.

Dans la forme de réalisation représentée, son contour est rectangulaire.

Soit L1 sa longueur et L2 sa largeur.

La longueur L1 est choisie pour correspondre au développé de l'ébauche de bandage 22′ recherchée.

La largeur L2 est choisie supérieure à la largeur L3 du dégagement 48 de la partie 40B du moule 40, tout en étant inférieure à la distance séparant l'un de l'autre les deux alignements de guides 42 que comporte celui-ci.

Initialement, la plaquette en céramique 33 ainsi constituée est simplement posée sur l'épaulement 44 de la partie 40B du moule 40, figure 11A.

Elle s'étend alors de part et d'autre de l'évidement 45 de cette partie 40B, entre les guides 42.

Dûment mise en place sur ces guides 42, la partie 40B du moule 40 vient simplement reposer par gravité sur la plaquette en céramique 33.

L'ensemble est alors disposé dans un four, sous vide, et la température de ce four est progressivement portée à 1500°C.

Conjointement, il est assuré un rapprochement relatif l'une par rapport à l'autre des deux parties 40A, 40B du moule 40, et, préférentiellement, ce rapprochement relatif se fait sous charge.

Dans la forme de mise en oeuvre représentée, les deux parties 40A, 40B étant disposées l'une au-dessus de l'autre, la partie 40A intervient très simplement par son propre poids pour le développement de la charge nécessaire.

Si désiré, et tel que schématisé en traits interrompus sur les figures 11A, 11B, 11C, elle peut cependant en sus être lestée par une masselotte 50.

Quoi qu'il en soit, lors de leur rapprochement relatif, les deux parties 40A, 40B du moule 40 se trouvent avantageusement guidées l'une par rapport à l'autre par les guides 42.

Dès 1300°C, et tel que schématisé à la figure 11B, il est observé un affaissement, par fluage, de la plaquette en céramique 33, qui, sous la sollicitation de la partie 40A du moule 40, se cintre progressivement, jusqu'à venir épouser la forme de l'évidement 45 de la partie 40B au terme du déplacement vers cette dernière de la partie 40A précédente, figure 11C.

La température est alors maintenue pendant un certain temps, par exemple pendant dix minutes.

La zircone ayant un coefficient de dilatation thermique supérieur à celui du graphite, l'ébauche de bandage 22′ ainsi obtenue peut avoir tendance, lors du refroidissement consécutif au chauffage précédent, à se refermer sur le bossage 47 de la partie 40A du moule 40, en pinçant alors celui-ci, au risque de se briser.

Ainsi qu'il est aisé de le comprendre, la dépouille d prévue entre ce bossage 47 et l'évidement 45 a précisément pour but de pallier les conséquences d'un tel pincement en donnant à la plaquette en céramique 33 tout le jeu nécessaire à cet effet.

Par exemple, cette dépouille d peut être de l'ordre 0,2 mm.

Conjointement, le dégagement 48 prévu dans la zone médiane de l'évidement 45 de la partie 40B du moule 40 permet avantageusement d'éviter que, par effet de bord, le cintrage auquel a été soumise longitudinalement la plaquette en céramique 33 s'accompagne également d'un cintrage transversal de celle-ci.

Le formage à chaud sous vide de la zircone constitutive de la plaquette en céramique 33 initiale se faisant en présence de graphite, matériau constitutif des deux parties 40A, 40B du moule 40, il s'accompagne inévitablement d'un noircissement de cette zircone.

Il suffit de reporter à nouveau à 1500°C, à l'air pur, l'ébauche de bandage 22′ obtenue pour que ce noircissement disparaisse.

Le procédé de réalisation d'un bandage en céramique 22 implique, ensuite, comme décrit ci-dessus, un usinage et un polissage de l'ébauche de bandage 22′ précédente.

Si désiré, et tel que schématisé en traits interrompus à la figure 12, le développé de cette ébauche de bandage 22′ peut être fait suffisant pour que celle-ci soit en mesure de se maintenir élastiquement, par simple encliquetage, sur la branche latérale 16 correspondante de la pièce fémorale 15.

De même, et tel qu'illustré par la figure 13, le moule 40 mis en oeuvre peut être apte à permettre le formage simultané, et d'un seul tenant, d'une ébauche de bandage 22˝ comportant deux branches 22′ correspondant chacune respectivement aux deux branches latérales 16 de la pièce fémorale 15 à équiper, avec, à la racine entre elles, une saignée 53 correspondant elle-même à la saignée 21 de cette pièce fémorale 15.

Ainsi, une partie au moins de cette saignée 21 est, elle aussi, munie d'un bandage en céramique.

Il en est de même dans la variante de réalisation représentée à la figure 14, suivant laquelle un bandage en céramique 52, distinct des bandages en céramique 22, est rapporté sur cette saignée 21.

Dans le cas d'une réalisation par formage, le développement de la charge nécessaire au formage pourrait en tout ou partie être assuré à l'aide d'un vérin.

Dans tous les cas, pour la réalisation d'un bandage en céramique préformé propre à l'équipement de la pièce fémorale concernée, ces procédés ont en commun de partir d'une pièce en céramique préexistante, de forme différente de la forme finale désirée, et de la conformer, en pratique à chaud, de manière à permettre son adaptation à la pièce fémorale à équiper.

Il est à souligner, en outre, que, par extension, le mot "céramique", ici utilisé par simple commodité, doit être entendu comme concernant par lui-même non seulement les céramiques proprement dites mais encore, d'une manière plus générale, tous les matériaux, et notamment les matériaux composites, tels que par exemple les matériaux carbone/carbone et les matériaux carbone/silicium, présentant des caractéristiques, et en particulier des caractéristiques de dureté, comparables, les matériaux composites, notamment, étant susceptibles de se prêter au polissage nécessaire après qu'il leur ait été appliqué un revêtement approprié, en carbone pyrolitique par exemple, même si, pris isolément, ils ne sont pas par eux-mêmes initialement aptes à un tel polissage.

Enfin, le domaine d'application de l'invention ne se limite pas non plus seulement au cas où, tel que plus particulièrement décrit et représenté, la pièce fémorale à équiper est en forme de fourche.

Au contraire, il s'étend tout aussi bien au cas où, s'agissant d'une prothèse unicompartimentale, cette pièce fémorale ne comporte qu'une seule branche.

## Revendications

1. Pièce fémorale pour prothèse du genou, du genre constituée d'une pièce métallique, caractérisée en ce qu'une partie au moins de sa surface frottante est munie d'un bandage en céramique (22, 52), ledit bandage en céramique constituant une pièce massive qui, initialement distincte d'elle, lui est dûment rapportée, en lui étant convenablement assujettie.

2. Pièce fémorale suivant la revendication 1, caractérisée en ce que, comportant, sous la forme générale d'une fourche globalement cintrée, d'une part, deux branches latérales (16), qui sont destinées à être chacune respectivement appliquées aux deux condyles du fémur à traiter, pour coopération avec les glènes du plateau tibial (14) correspondant, et, d'autre part, une partie médiane (20), avec laquelle se raccordent les branches latérales (16) précédentes, chacune de ses branches latérales (16) est munie d'un bandage en céramique (22) sur une partie au moins de sa longueur.

3. Pièce fémorale suivant la revendication 2, caractérisée en ce que le bandage en céramique (22) de chacune de ses branches latérales (16) est en zircone.

4. Pièce fémorale suivant l'une quelconque des revendications 2, 3, caractérisée en ce que le bandage en céramique (22) de chacune de ses branches latérales (16) s'étend sur la totalité de la longueur utile de celle-ci.

5. Pièce fémorale suivant l'une quelconque des revendications 2 à 4, caractérisée en ce que le bandage en céramique (22) de chacune de ses branches latérales (16) va en diminuant de largeur de la partie postérieure vers la partie antérieure.

6. Pièce fémorale suivant l'une quelconque des revendications 2 à 5, caractérisée en ce que le bandage en céramique (22) de chacune de ses branches latérales (16) est assujetti à celle-ci par sertissage ou collage.

7. Pièce fémorale suivant l'une quelconque des revendications 2 à 6, caractérisée en ce que, entre chacune de ses branches latérales (16) et le bandage en céramique (22) que porte celle-ci, sont interposés des moyens d'amortissement (25).

8. Pièce fémorale suivant la revendication 7, caractérisée en ce que, sa partie médiane (20) présentant une saignée (21) formant trochlée, ladite saignée (21) est également munie d'un bandage en céramique (52) sur une partie au moins de sa surface.

## Claims

1. Femoral piece for a knee prosthesis, of the type consisting of a metal piece, characterised in that at least part of its friction surface is provided with a ceramic band (22, 52), the said ceramic band forming a solid piece which, initially separate from it, is duly attached to it, by being suitably secured to it.

2. Femoral piece according to Claim 1, characterised in that, including, in the general form of an overall arched fork, on the one hand two lateral arms (16), which are designed each to be applied respectively to the two condyles of the femur to be treated, to cooperate with the sockets of the corresponding tibial plate (14), and on the other hand a middle part (20), with which the aforesaid lateral arms (16) connect, each of its lateral arms (16) is provided with a ceramic band (22) over at least part of its length.

3. Femoral piece according to Claim 2, characterised in that the ceramic band (22) of each of its lateral arms (16) is made from zirconia.

4. Femoral piece according to either one of Claims 2 or 3, characterised in that the ceramic band (22) of each of its lateral arms (16) extends over the entire useable length of the latter.

5. Femoral piece according to any one of Claims 2 to 4, characterised in that the width of the ceramic band (22) of each of its lateral arms (16) decreases from the rear part towards the front part.

6. Femoral piece according to any one of Claims 2 to 5, characterised in that the ceramic band (22) of each of its lateral arms (16) is secured to the latter by crimping or bonding.

7. Femoral piece according to any one of Claims 2 to 6, characterised in that damping means (25) are interposed between each of its lateral arms (16) and the ceramic band (22) carried by it.

8. Femoral piece according to Claim 7, characterised in that, its middle part (20) having a groove (21) forming a trochlea, the said groove (21) is also provided with a ceramic band (52) over at least part of its surface.

## Patentansprüche

1. Femorales Teil für eine Knieprothese, welches aus einem Metallteil gebildet ist, dadurch gekennzeichnet, daß zumindest ein Teil seiner reibenden Oberfläche mit einer Bandage aus Keramik (22, 52) versehen ist, wobei die Bandage aus Keramik ein massives Teil bildet, welches, anfänglich von ihm getrennt mit ihm in geeigneter Weise verbunden ist, wobei es ihm in geeigneter Weise zugeordnet ist.

2. Femorales Teil gemäß Anspruch 1, dadurch gekennzeichnet, daß es in seiner generellen Form eine Gabel darstellt,welche global gekrümmt ist, mit einerseits zwei lateralen Ästen (16), welche dazu bestimmt sind, jeweils auf zwei Knochengelenken des zu behandelnden Oberschenkels angewendet zu werden, und zwar zum Zusammenwirken mit den Knochengelenkvertiefungen der entsprechenden, zum Schienbein gehörenden Ebene (14), und andererseits mit einem medianen Abschnitt (20), mit welchem sich die vorangegangenen lateralen Äste (16) verbinden, wobei jeder dieser lateralen Äste (16) mit einer Bandage aus Keramik (22) zumindest auf einem Abschnitt seiner Länge versehen ist.

3. Femorales Teil gemäß Anspruch 2, dadurch gekennzeichnet, daß die Bandage aus Keramik (22) von jedem seiner lateralen Äste (16) aus Zirkonium ist.

4. Femorales Teil gemäß einem der Ansprüche 2 oder 3, dadurch gekennzeichnet, daß die Bandage aus Keramik (22) von jedem seiner lateralen Äste (16) sich über die Gesamtheit der Nutzlänge von diesen erstreckt.

5. Femorales Teil gemäß einem der Ansprüche 2 bis 4, dadurch gekennzeichnet, daß die Bandage aus Keramik (22) von jedem seiner lateralen Äste (16) sich in der Breite verjüngt, und zwar von dem hinteren Abschnitt zu dem vorderen Abschnitt.

6. Femorales Teil gemäß einem der Ansprüche 2 bis 5, dadurch gekennzeichnet, daß die Bandage aus Keramik (22) mit jedem seiner lateralen Äste (16) mittels Bördel-Verbindung oder Verklebung verbunden ist.

7. Femorales Teil gemäß einem der Ansprüche 2 bis 6, dadurch gekennzeichnet, daß zwischen jedem seiner lateralen Äste (16) und der Bandage aus Keramik (22), welche dieser trägt, Vorrichtungen zum Dämpfen (25) zwischengelagert sind.

8. Femorales Teil gemäß Anspruch 7, dadurch gekennzeichnet, daß sein medianer Abschnitt (20) einen Einschnitt (21) aufweist und eine Trochlea bildet, wobei der Einschnitt (21) auf einem Abschnitt seiner Oberfläche ebenfalls mit einer Bandage aus Keramik (52) versehen ist.
